# EUROPEAN PATENT SPECIFICATION

(11) **EP 1 729 702 B1**
(45) Date of publication and mention of the grant of the patent: **11.09.2013**
(21) Application number: 05722228.3
(22) Date of filing: 16.03.2005
(51) Int. Cl.: A61F 7/12

(54) **CEREBRAL TEMPERATURE CONTROL**
ZEREBRALE TEMPERATURKONTROLLE
CONTROLE DE LA TEMPERATURE CEREBRALE

(30) Priority: 16.03.2004 US 708624
(43) Date of publication of application: 13.12.2006
(73) Proprietor: Quickcool AB, 223 70 Lund (SE)
(72) Inventor: LUNDERQVIST, Anders, S-226 39 Lund (SE); ALLERS, Mats, S-226 50 Lund (SE); BORIS-MÖLLER, Fredrik, S-223 51 Lund (SE); WIELOCH, Tadeusz, S-222 22 Lund (SE)
(74) Representative: Asketorp, Göran
(86) International application number: PCT/SE2005/000382
(87) International publication number: WO 2005/087156

(56) References cited:
- WO-A1-98/23217
- DE-A1- 19 952 440
- US-A- 3 766 924
- US-A- 5 792 100
- US-A1- 2002 138 121
- US-A1- 2002 161 349
- US-A1- 2004 049 154

## Description

### Technical field of the invention

The present invention refers to devices for the control of the temperature of the brain of a human being, and especially to cooling of the brain.

### Background of the invention

In pathological conditions, the body temperature or the temperature of body parts cf a human being influences the healing process and the risk of permanent damage. Cancer cells, for example, are heat sensitive and a local heating of the blood flow around a cancer tumor may for some types of cancer constitute a treatment resulting in restrained tumor growth or in some cases even in a shrinking of the tumor. In other cases cooling of a body part may be important to reduce adverse secondary symptoms of the pathological condition, this is primarily intended for the treatment of brain ischemia.

In the case of a stroke, the blood flow to the brain is reduced (ischemia) due to a hemorrhage or the clogging of a blood vessel. This condition will cause permanent functional deficits unless treatment to restore blood flow and protect nerve cells is initiated at an early stage, which will reduce the loss of bodily functions, such as paralysis. It is well known that cooling the brain effectively blocks the development of cellular damage after an episode of ischemia.

Whole body cooling of the patient suffering transient circulatory arrest to the brain results in a reduction of the symptoms of neurological deficit. However, there are certain problems associated with whole body cooling. One is that the cooling is not fast enough to effectively use its protective potential. Another problem is that whole body cooling must be carried out under close control of physic-logical parameters or under anesthesia. Yet another problem is that there is a risk of cardiovascular complications.

In the case of a circulatory arrest, the brain can suffer permanent damage if the arrest exceeds a time period of about 5-15 minutes. However, if the temperature of the brain is lowered before, during or after the arrest, the brain damage is diminished.

In the case of brain trauma the brain suffers from open or close head concussion. Hypothermia has been shown to diminish traumatic brain injury in such cases.

There are several methods in the prior art to carry out a more isolated cooling of a single organ or body part. An example of cooling of the brain in a human being is disclosed in the patent document WO 98/23217, relating to a method of cerebral retro-perfusion and retro-infusion, involving the cooling of arterial blood that then is returned to the entire brain. However, this method entails a large and complicated surgical procedure, which delays the onset of an actual treatment.

US 5,906,588 discloses a method and a device for heart-lung bypass and cooling of a specific body part. This disclosure primarily relates to complicated heart surgery and organ transplantation.

The patent document US 2002/0161349 A1 discloses a first phase comprising the step of introducing an infusion catheter for infusion of a temperature controlled infusion solution or perfusate into a vein initiating quick general body hypothermia. An optional second phase may be used, wherein a second infusion catheter is introduced into an artery of the human being, the second infusion catheter being configured to provide selective temperature control of the brain and infusion of other important substrates and pharmacological compounds into the brain. A third phase is also disclosed, wherein an extra-corporeal circuit is established between a vein and an artery. Blood is withdrawn from the vein, and the temperature of the blood is modified outside the body and the blood is returned to the body through the artery.

In many animals, the brain can be protected from overheating by heat exchange over the rete mirabile at the base of the brain. Here a fine network of the carotid artery is in close connection to a similar network of veins draining the brain. Respiratory evaporation from the surfaces of the nostrils and the epipharynx cools veins in this region flowing towards the rete mirabile whereby heat exchange can occur between arteries and veins. In animals without a rete mirabile, a variation of more or less complicated scrolls of conchae in the nasal cavity enhances evaporation and the heat exchange.

Flushing the nasal cavities with fluids have been shown to reduce the brain temperature (Natale J E et al Stroke 1989; 20:770-777, Hagioka S et al. Crit. Care. Med. 2003; 31:2502-2508). To prevent the flushing fluid to enter the trachea, however, tracheostomy or intubations are necessary and still it would be difficult to prevent fluid to enter the esophagus.

Document DE 19952440 A1 discloses a cooling device suited to be introduced into the sinus sphenoidalis, which is a space arranged in close proximity to the brain tissue and is accessible via the nose or via the nostrils. This can be accomplished in particular after performing a first computer tomography, since a sufficient level of knowledge is then present over respective anatomical conditions and the possible access roads. Then, the cooling device can be inserted for example by proven endoscopic methods and equipment.

Document US 2002/0138121 A1 discloses a method of treating cancer and other diseases by modulating body temperature. Heat may directed to the hypothalamus of a warm-blooded animal to cool the animal, utilizing the physiological mechanisms that regulate body temperature to effect a compensatory cooling response, thereby lowering body temperature (hypothermia), and rendering other methods of lowering body temperature more effective. Heat may be withdrawn from the hypothalamus of an animal, cooling the hypothalamus, inducing a compensatory increase in body temperature (hyperthermia), and rendering other methods of raising body temperature more effective. Body temperature may be directly modulated by heat-exchange catheter positioned within a blood vessel of a patient. The device relates generally to methods of treating cancer by inducing hypothermia by directing heat to the hypothalamus, optionally maintaining cancerous tissue at or near to normal body temperature, and optionally applying another cancer treatment. This other cancer treatment may be radiation therapy, chemotherapy, a combination of radiation and chemotherapy, or some other cancer treatment. The invention relates generally to methods of treating diseases including cancer, viral infections, and other diseases, comprising inducing hyperthermia by cooling the hypothalamus, and optionally applying another treatment, for example radiation, chemotherapy, antiviral therapy, or a combination of therapies.

Among others, there is disclosed a double lumen catheter for circulating heating fluid, the catheter being suited for insertion into the sphenoid sinus. A distendible balloon is arranged at the tip of said catheter, which balloon can expand under the positive pressure head following partial occlusion of the outflow.

### Disclosure of the invention

The present invention is based on the discovery that the cerebral arteries can temper the human brain by remnants of the venous carotid rete, i.e. the cavernous and other sinuses, surrounding the carotid and other cerebral arteries, such as vertebral arteries, before entering the brain. Cold air or fluids in the nasal cavities including the epipharynx are in close thermal contact with the veins draining to the dense venous plexus surrounding the internal carotid and vertebral arteries and to the cavernous sinus. Moreover, the sub mucosal position of the carotid arteries in the epipharynx might play an additional role in this heat exchange.

The hypothesis is supported by Mariak Z et al. (J. Appl Physiol 1999; 87:1609-13) who demonstrated a slight fall of the brain temperature when intubated patients (no cold venous blood from nasal cavities) were extubated and were allowed to breath normally and this in spite of the poor heat conduction of air.

According to the present invention, a device for selective hypothermia treatment of the brain in patients with brain ischemia, such as stroke and cardiac arrest, is disclosed.

An object of the present invention is to provide a system for quick and efficient control of the temperature of the brain without substantially changing the temperature of the rest of the body.

Another object of the invention is to provide a system that is simple and initially does not require specialized personnel acquainted with for example radiology or other diagnostic imaging techniques.

In a first aspect of the invention, there is provided a system for indirect temperature regulation by cooling of the brain of a human being via a nasal cavity thereof, comprising a double lumen catheter means suited to be introduced through a nostril of a human being and placed with its tip adjacent the back of said nasal cavities, said double lumen catheter means comprising a first lumen and a second lumen, said first and second lumens being in fluid communication by means of a set of openings, said second lumen forming a membrane suited to be arranged in contact with a surface of said nasal cavity, said membrane defining an expandable balloon; an inlet means for introducing cooling fluid into said expandable balloon; an outlet means for removing cooling fluid frcm said expandable balloon; and means for circulating said cooling fluid into said expandable balloon via said inlet means and out of said expandable balloon via said outlet means said means comprising a set of tubings configured to connect said catheter means to a reservoir; said inlet means being in fluid communication with said reservoir and with said first lumen, said inlet means being configured to receive said fluid from said reservoir, and said outlet means being in fluid communication with said second lumen and with said reservoir; said balloon having a size so that, when inflated, it is expandable to cover the inner surface of the nasal cavity, and further comprising another double lumen catheter means suited to be introduced through another nostril of the human being and placed with its tip adjacent the back of said nasal cavities, said another double lumen catheter means comprising a third lumen and a fourth lumen, said third and fourth lumens being in fluid communication by means of another set of openings, said fourth lumen forming a membrane suited to be arranged in contact with another surface of said nasal cavity, said membrane defining another expandable balloon; another inlet means for introducing cooling fluid into said expandable balloon; another outlet means for removing cooling fluid from said expandable balloon; and another means for circulating said cooling fluid into said expandable balloon via said another inlet means and out of said expandable balloon via said another outlet means said another means comprising another set of tubings configured to connect said another catheter means to the reservoir, said another inlet means being in fluid communication with said reservoir and with said third lumen, said inlet means being configured to receive said fluid from said reservoir, and said another outlet means being in fluid communication with said fourth lumen and with said reservoir, said another expandable balloon having a size so that, when inflated, it is expandable to cover the inner surface of the nasal cavity, and further comprising pressure regulating nozzles arranged at said tubings adjacent said reservoir, said pressure regulating nozzles being configured to provide resistance in said tubings.

In an embodiment, the system may comprise a temperature regulator connected to said reservoir, said regulator being configured to regulate the temperature of said fluid, via a cooling device. Furthermore, said inlet means and outlet means may be arranged at a proximal portion of the catheter means.

In a further embodiment, the circulation of said fluid may be accomplished by means of hydrostatic pressure of said fluid in said reservoir. Alternatively, the means for circulating said fluid may comprise a pumping means arranged between said reservoir and said catheter means. The means for circulating the fluid may be configured to provide a flow rate of approximately 25 - 1000 ml/min.

In another embodiment, the pressure regulating nozzles may be variable.

In a still further embodiment, the system may further comprise a temperature monitoring device configured to, indirectly or directly, register the temperature of the brain and to automatically control said temperature regulator to regulate the temperature of said fluid in said reservoir, via a heat exchanging device, in order to maintain the temperature of the brain at a desired level. The temperature monitoring device may be an IR thermistor, a MR device, a Near Infra Red device or an impedance spectroscopy device. The temperature monitoring device may be arranged on a forehead of the human being or in an auditory canal of the human being. The temperature of the brain may be approximately 10 - 41° C, such as 20 - 35° C.

### Brief description of the drawings

Further objects, features and advantages will become evident from the following description of several embodiments of the invention with reference to the enclosed drawings, in which:
Fig 1 is a schematic view of an embodiment of a single system;
Fig 2 is a schematic view of an embodiment of a system according to the present invention;
Fig 3 is a cross-sectional view of the double catheter means arranged at the inner surface of the nose, the nasal cavity, and epipharynx of a patient;
Fig 4 is a diagram showing an example of the registered temperature variation in the brain as a function of the time, when the inventive system is applied to a laboratory animal in the form of a pig;

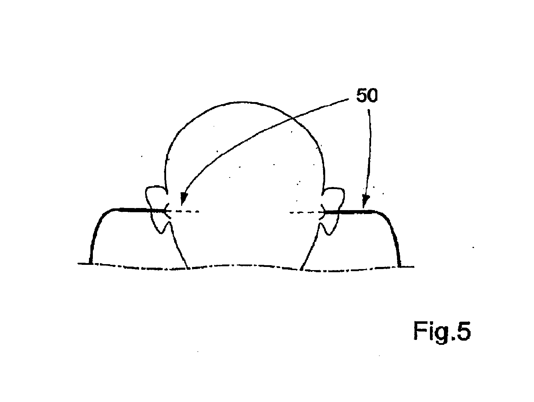

Fig 5 is a schematic view of two temperature sensors, each of which is arranged in an auditory channel;
Fig 6 is a diagram of different combinations of cooling techniques.

### Detailed description of the invention

The present invention will now be described with reference to the accompanying drawings.

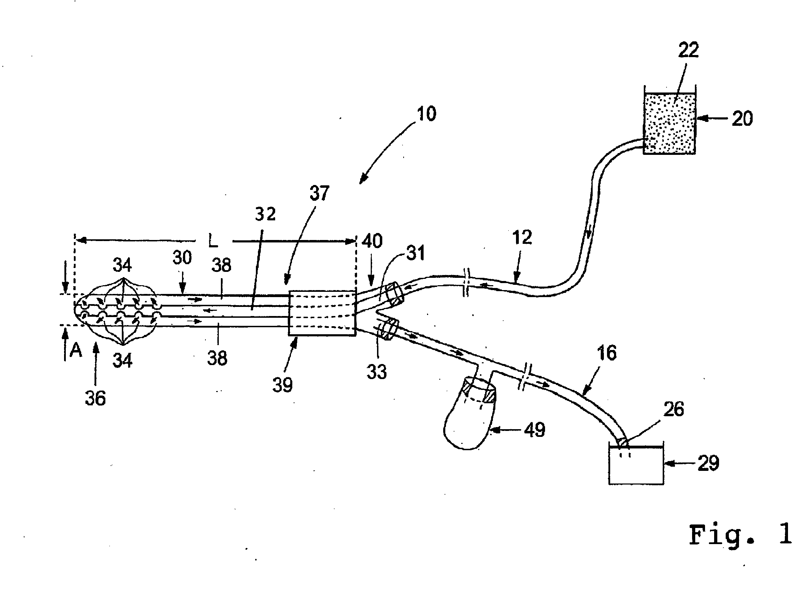

Fig 1 discloses an embodiment of a single system 1C for cerebral temperature control of a human being, in which the system comprises one double lumen catheter means 30.

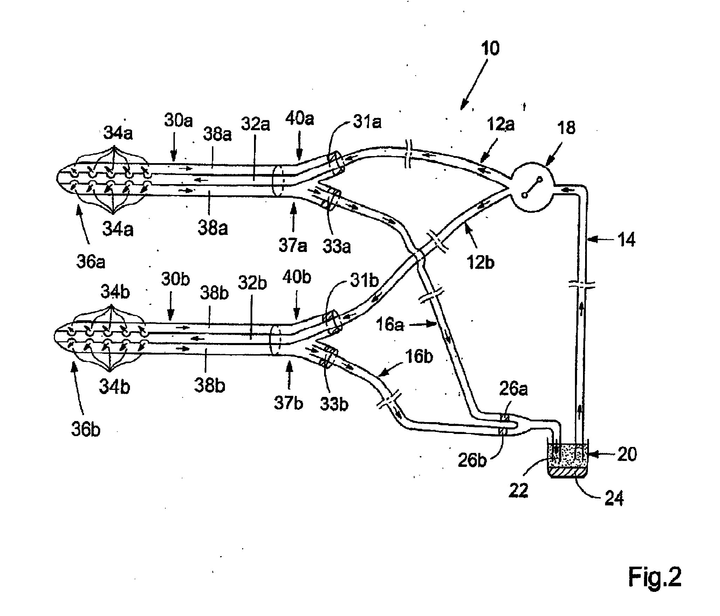

Fig 2 shows schematically a second embodiment of the inventive system, which system comprises two double lumen catheters means 30a, 30b to be introduced in respective nostril of a human being. By means of two catheter means, temperature regulation may be accomplished more effectively and quicker.

In the figures, the same reference numerals are used to indicate corresponding components. Further, in Fig 2, the corresponding components and details of the respective catheter means are distinguished by means of the addition of the letter a respective b to the reference numeral. Arrows are used to show the flow direction of the fluid in the system. However, as understood by the skilled person, the fluid flow can have the opposite direction to that indicated in the drawings.

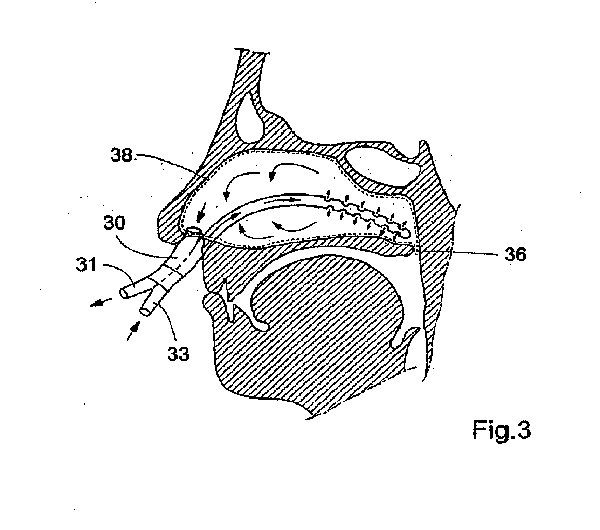

The system 10 comprises a double lumen balloon catheter means 30, 30a, 30b configured to be introduced through a nostril of a human being and to be placed with its tip at the level of the back of the nasal cavities, as shown in Fig 3. Pushing a sleeve or sheath 39 backward or forward may vary the length of double lumen balloon catheter.

The double lumen catheter means 30, 30a, 30b comprises a first lumen 32, 32a, 32b and a second lumen 38, 38a, 38b. The first and second lumens are arranged in fluid communication with each other by means of a set of end openings 34, 34a, and 34b. The second lumen 38, 38a, 38b is configured as an expandable balloon.

The system 10 could also comprise a temperature regulator 24, shown in Fig 2, connected to a reservoir 20 comprising a fluid 22. The temperature regulator 24 is configured to regulate the temperature of the fluid 22 comprised in the reservoir. The fluid 22 is for example a saline solution. The temperature regulator controls a heat exchanging device, namely a cooling device, for temperature regulating the fluid.

Means are arranged in the form of tubes 12, 12a, 12b, 14, 16, 16a, 16b for circulating said temperature regulated fluid 22 from the reservoir 20 to the catheter means 30, 30a, and 30b. The means 12, 12a, 12b, 14, 16, 16a, 16b are configured to circulate the fluid 22 into the first lumen 32, 32a, 32b of the catheter means 30, 30a, 30b, from the first lumen 32, 32a, 32b into the second lumen 38, 38a, 38b, and from the second lumen 38, 38a, 38b back to said reservoir 20, or vice versa.

The circulation means 12, 12a, 12b, 14, 16, 16a, 16b for circulating said temperature regulated fluid 22 comprises a set of tubings 12, 12a, 12b, 14, 16, 16a, 16b configured to connect the catheter means 30, 30a, 30b to the reservoir 20.

In use, when the fluid enters the second catheter 38, 38a, 38b, the second catheter 38, 38a, 38b is expanded more or less as a balloon to completely or almost completely cover the inner surface of the nose and epipharynx, as shown in Fig 3, whereby the naso-pharyngeal membranes may be temperature regulated. The temperature is transferred to adjacent arterial and venous structures, which are transferring the temperature to the brain.

Further, by means of the inventive arrangement the temperature regulated fluid 22 circulates in a closed fluid system 12, 12a, 12b; 14; 16, 16a, 16b; 30a, 30b; 20.

The double lumen catheter means 30, 30a, 30b comprises an inlet 31, 31a, 31b arranged to provide fluid communication with the reservoir 20 and with the first lumen 32, 32a, and 32b. The inlet 31, 31a, 31b is further configured to receive an amount of the temperature regulated fluid 22 from the reservoir 20. The first lumen 32, 32a, 32b has a set of distal end openings 34, 34a, 34b in a front-end portion 36, 36a, 36b of the catheter means 30, 30a, and 30b. The end openings 34, 34a, 34b being arranged to provide fluid communication with said second lumen 38, 38a, and 38b. The catheter means 30, 30a, 30b further comprises an outlet 33, 33a, 33b configured to provide fluid communication with the second lumen 38, 38a, 38b and with the reservoir 20.

The inlet 31, 31a, 31b and the outlet 33, 33a, 33b are arranged at an end portion 40, 40a, 40b of the catheter means 30, 30a, 30b.

According to an embodiment, the second lumen 38, 38a, 38b has a variable length L of approximately 5 - 25 cm. The sleeve 39 around the shaft of the catheter can reduce the length L of the second lumen when the sleeve is moved backward or forward. In expanded state, the diameter A of the second lumen at a front part 36, 36a, 36b of the second lumen is approximately 2 - 4 cm and the diameter B of the second lumen at a base part 37, 37a, 37b of the second lumen is approximately 1 - 3 cm, but these measures can vary if the balloon pressure is increased.

The catheter means 30, 30a, 30b could be manufactured of a flexible material such as plastic, synthetic latex, silicone or Gore-Tex. The material may be coated with a substance resulting in a hydrophilic surface. An anesthetic agent may also cover the surface.

According to an embodiment of the invention, the circulation of the fluid 22 is accomplished by means of the hydrostatic pressure of the fluid 22 in the reservoir 20. As shown in Fig 1, the reservoir 20 is arranged at a level, which is higher than the nasal cavity of the human being under treatment, and a waste bag 29 is arranged below the same level. Thence, the cold fluid flows by gravity from the reservoir and to the catheter device and further to the waste bag.

However, according to another embodiment of the invention, the means 12, 14, 16 for circulating the temperature regulated fluid 22 further comprises a pumping means 18 arranged between the reservoir 20 and the catheter means 30, 30a, 30b by means of tubings 12, 12a, 12b, 14. Thus, it should be understood that the pumping means 18 illustrated in Fig 2 is optional.

The circulation means for circulating the fluid is preferably configured to provided a fluid flow rate of 25 - 1000 ml/min.

The system can also comprise a pressure regulating nozzle 26, 26a, 26b, shown in Figs 1 and 2, arranged at the tubings 16, 16a, and 16b. The pressure regulating nozzle 26, 26a, 26b is arranged close to the reservoir 20 or the waste bag 29 and being configured to provide a resistance in the tubings, whereby the fluid flowing in the system is able to expand the second lumen 38, 38a, 38b. The nozzle may be a clamp that is adjustable.

Moreover, pressure sensors 49 may be arranged for measuring the pressure in the second lumen, as shown in Fig 1. Such a pressure sensor may be a similar construction as the second lumen arranged in a side branch. By means of the pressure sensor, it can be assured that the pressure will not be too high, which may be experienced Lo be uncomfortable. The pressure sensor may as well be conventional pressure meters.

The technique according to the present invention involves the following, as shown in Fig 3. Two double lumen thin walled balloon catheters are inserted into the nostrils of the patient. When inflated and circulated with fluid, e.g. saline, at a temperature of ±0 to 41°C, preferably 20-35°C, at a rate of 25 to 1000 ml per min, the lower range for neonate, the balloons will cover the inner surfaces of the nose and the epipharynx, collectively named the nasal cavities. Circulation can be performed in either direction. Cooling of the nasal cavities can then be accomplished without any fluids entering the trachea or the esophagus. Different sizes of catheters and balloons will fit neonates to adults and a sheath or sleeve around the shaft of the catheter can reduce the length of the ballcon when the sheath is moved forward.

The system can also comprise a temperature sensor 50, which is connectable to the temperature regulator 24 and configured to register or estimate the temperature of the brain and to automatically control the temperature regulator 24 to regulate the temperature of the fluid in the reservoir 20 in order to maintain the temperature of the brain at a desired level. The temperature sensor is for example an IR thermistor, which could be arranged in an auditory canal of the human being or on the skin, such as the forehead, of the human being. The temperature could also be monitored by a MR device, a Near Infrared device or an impedance spectroscopy device. The desired brain temperature level may be approximately 10 - 35°C. The temperature level depends on the degree of hypothermia, which is clinically relevant. If the brain temperature is increased it should be closely monitored.

The present invention also relates to a kit of disposables for use in the inventive system. The kit comprises a plurality of tubings, and the double lumen catheter means 30, 30a, 30b.

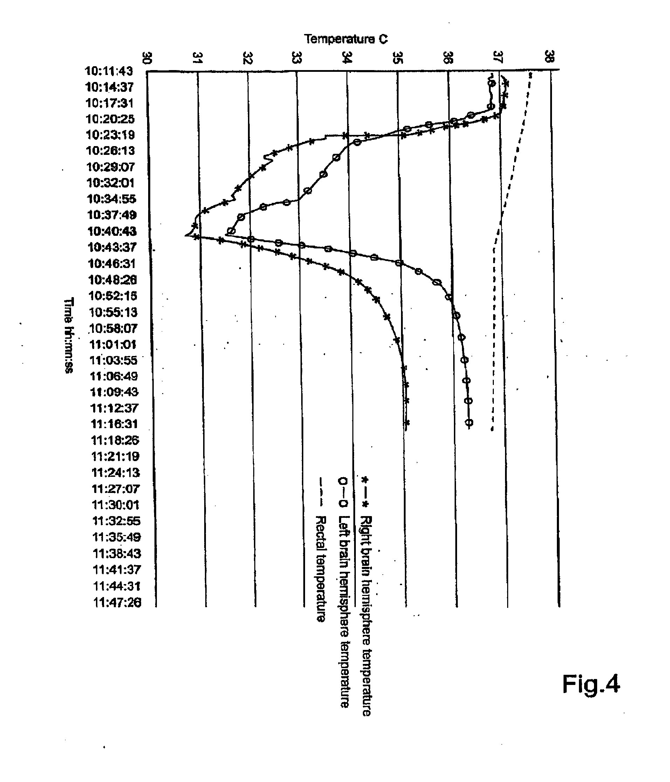

Fig 4 schematically shows how the registered temperature in the left and right brain hemisphere is reduced after the onset of the inventive method, while the registered temperature in the rectum remains almost unchanged. In the performed experiment, a double lumen catheter means was introduced in respective nostril of an experimental animal. The temperature regulator controls the fluid temperature to desired level through a cooling element. Circulation of cold fluid started approximately at 10:18 and stopped approximately at 10:41 (after 23 minutes). As seen from Fig 4 the registered temperature in the left and right brain hemisphere is reduced within a short period of time after the start and increases again after the stop. Further, the temperature of the rectum is almost unchanged. Thus, a selectively cooling of the brain is accomplished.

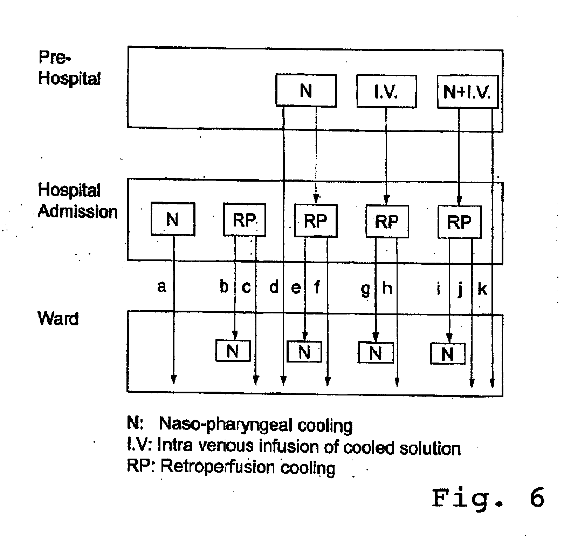

The introduction of balloon catheters into the nostrils of the patient will make rapid selective brain cooling possible. At the arrival into the hospital admission, suitable alternatives (see Fig 6) for the continuation of the hypothermia treatment are decided according to the condition of the patient.

Paramedic's start at the site an intravenous infusion of ice cooled lactated Ringers solution, possibly combined with naso-pharyngeal cooling or the treatment starts with naso-pharyngeal cooling alone.

Depending on the condition of the patient at the arrival to the hospital the following alternatives for future treatment can be seen in the admission flow chart according to Fig 6.

Patients with naso-pharyngeal cooling or retro perfusion treatment at the admission unit are later referred to the ward for future treatment according to Fig 6.

According to the invention, the tissue in the nose and epipharynx are cooled, via a membrane in contact with the tissue. In the present specification and claims, the expression "nasal cavity" is used to mean all inside surface of the nose (via both nostrils) and including the epipharynx. In some embodiments, only a portion of the nasal cavity surfaces is used for cooling purpose, and this is intended to be within the scope of the invention.

The cooling effect of the fluid can be monitored by measuring the inlet and outlet temperature of the fluid as well as the flow rate. By calorimetric calculations, the cooling effect can be calculated. If it is presumed that the heat generation of the brain or tissue inside the head is relatively constant, the temperature decrease can be calculated or predicted. Furthermore, temperature measurements at different places of the body and with different methods as indicated above, can provide further data for a closer prediction of the brain temperature.

The intention of the invention is to cool substantially only the brain, including adjacent tissue, but maintain the temperature of the rest of the body at normal temperature. It may be that the body temperature may also be lowered and this tendency may be counteracted by covering part of the body by a blanket or by providing slightly heated warm air.

The present invention has been described with reference to embodiments and an example. However, it should be understood that modifications of components or functional steps could be performed without deviating from the scope of the invention.

## Claims

1. A system for indirect temperature regulation by cooling of the brain of a human being via a nasal cavity thereof, comprising
a double lumen catheter means (30a) suited to be introduced through a nostril of a human being and placed with its tip adjacent the back of said nasal cavities,
said double lumen catheter means comprising a first lumen (32a) and a second lumen (38a), said first and second lumens being in fluid communication by means of a set of openings (34a), said second lumen forming a membrane suited to be arranged in contact with a surface of said nasal cavity, said membrane defining an expandable balloon;
an inlet means for introducing cooling fluid into said expandable balloon;
an outlet means for removing cooling fluid from said expandable balloon; and
means for circulating said cooling fluid into said expandable balloon via said inlet means and out of said expandable balloon via said outlet means said means comprising a set of tubings (12a, 16a) configured to connect said catheter means to a reservoir (20),
said inlet means being in fluid communication with said reservoir (20) and with said first lumen (32a), said inlet means being configured to receive said fluid from said reservoir, and said outlet means being in fluid communication with said second lumen (38a) and with said reservoir (20),
said balloon having a size so that, when inflated, it is expandable to cover the inner surface of the nasal cavity., and further comprising
another double lumen catheter means (30b) suited to be introduced through another nostril of the human being and placed with its tip adjacent the back of said nasal cavities,
said another double lumen catheter means (30b) comprising a third lumen (32b) and a fourth lumen (38b), said third and fourth lumens being in fluid communication by means of another set of openings (34b), said fourth lumen forming a membrane suited to be arranged in contact with another surface of said nasal cavity, said membrane defining another expandable balloon;
another inlet means for introducing cooling fluid into said expandable balloon;
another outlet means for removing cooling fluid from said expandable balloon; and another means for circulating said cooling fluid into said expandable balloon via said another inlet means and out of said expandable balloon via said another outlet means said another means comprising another set of tubings (12b, 16b) configured to connect said another catheter means to the reservoir (20),
said another inlet means being in fluid communication with said reservoir (20) and with said third lumen (32b), said inlet means being configured to receive said fluid from said reservoir, and said another outlet means being in fluid communication with said fourth lumen (38b) and with said reservoir (20),
said another expandable balloon having a size so that, when inflated, it is expandable to cover the inner surface of the nasal cavity,
and further comprising
pressure regulating nozzles (26a, 26b) arranged at said tubings (16a, 16b) of the outlet means adjacent said reservoir, said pressure regulating nozzles being configured to provide resistance in said tubings (16a, 16b) of the outlet means.

2. The system according to claim 1, wherein a temperature regulator (24) is connected to said reservoir, said regulator being configured to regulate the temperature of said fluid, via a cooling device.

3. The system of claim 1, wherein said inlet means and outlet means are arranged at a proximal portion (40,40a, 40b) of the catheter means.

4. The system of any of the previous claims, wherein said circulation of said fluid is accomplished by means of hydrostatic pressure of said fluid in said reservoir.

5. The system of any of claims 1 to 3, wherein said means for circulating said fluid further comprises a pumping means (18) arranged between said reservoir and said catheter means.

6. The system of any of the previous claims, wherein said means for circulating the fluid is configured to provide a flow rate of approximately 25 - 1000 ml/min.

7. The system of of any of the previous claims, wherein, said pressure regulating nozzles are variable.

8. The system of claim 1, further comprising a temperature monitoring device (50) configured to, indirectly or directly, register the temperature of the brain and to automatically control said temperature regulator to regulate the temperature of said fluid in said reservoir, via a heat exchanging device, in order to maintain the temperature of the brain at a desired level.

9. The system of claim 8, wherein said temperature monitoring device is an I R termistor, a MR device, a Near Infra Red device or an impedance spectroscopy device.

10. The system of claim 8 or 9, wherein said temperature monitoring device is suited to be arranged on a forehead of the human being or in an auditory canal of the human being.

## Patentansprüche

1. System zur indirekten Temperaturregelung durch Kühlen des Gehirns eines Menschen über eine Nasenhöhle desselben, umfassend
ein Doppellumenkathetermittel (30a), das dafür geeignet ist, durch das Nasenloch eines Menschen eingeführt und mit seiner Spitze angrenzend an die Rückseite der Nasenhöhlen platziert zu werden,
wobei das Doppellumenkathetermittel ein erstes Lumen (32a) und ein zweites Lumen (38a) umfasst, wobei das erste und zweite Lumen über ein Fluid mittels einer Gruppe von Öffnungen (34a) in Verbindung stehen, wobei das zweite Lumen eine Membran bildet, die dafür geeignet ist, eine Fläche der Nasenhöhle berührend angeordnet zu werden, wobei die Membran einen dehnbaren Ballon definiert;
ein Einlassmittel zum Einbringen von Kühlfluid in den dehnbaren Ballon;
ein Auslassmittel zum Abführen von Kühlfluid aus dem dehnbaren Ballon; und
Mittel zum Zirkulierenlassen des Kühlfluids in den dehnbaren Ballon über das Einlassmittel und aus dem dehnbaren Ballon über das Auslassmittel heraus, wobei die Mittel eine Gruppe von Röhren (12a, 16a) umfassen, die so eingerichtet sind, dass sie das Kathetermittel mit einem Behälter (20) verbinden,
wobei das Einlassmittel über ein Fluid mit dem Behälter (20) und mit dem ersten Lumen (32a) in Verbindung steht, wobei das Einlassmittel so eingerichtet ist, dass es das Fluid aus dem Behälter aufnimmt, und das Auslassmittel mit dem zweiten Lumen (38a) und mit dem Behälter (20) über ein Fluid in Verbindung steht,
wobei der Ballon so groß ist, dass er, wenn er aufgeblasen wird, dehnbar ist, sodass er die Innenfläche der Nasenhöhle bedeckt, und ferner umfassend
ein weiteres Doppellumenkathetermittel (30b), das dafür geeignet ist, durch ein anderes Nasenloch eines Menschen eingeführt und mit seiner Spitze angrenzend an die Rückseite der Nasenhöhlen platziert zu werden,
wobei das weitere Doppellumenkathetermittel (30b) ein drittes Lumen (32b) und ein viertes Lumen (38b) umfasst, wobei das dritte und vierte Lumen über ein Fluid mittels einer weiteren Gruppe von Öffnungen (34b) in Verbindung stehen, wobei das vierte Lumen eine Membran bildet, die dafür geeignet ist, eine weitere Fläche der Nasenhöhle berührend angeordnet zu werden, wobei die Membran einen weiteren dehnbaren Ballon definiert;
ein weiteres Einlassmittel zum Einbringen von Kühlfluid in den dehnbaren Ballon;
ein weiteres Auslassmittel zum Abführen von Kühlfluid aus dem dehnbaren Ballon; und
ein weiteres Mittel zum Zirkulierenlassen von Kühlfluid in den dehnbaren Ballon über das weitere Einlassmittel und aus dem dehnbaren Ballon über das weitere Auslassmittel heraus, wobei das weitere Mittel eine weitere Gruppe von Röhren (12b, 16b) umfasst, die so eingerichtet sind, dass sie ein weiteres Kathetermittel mit dem Behälter (20) verbinden,
wobei das weitere Einlassmittel über ein Fluid mit dem Behälter (20) und mit dem dritten Lumen (32b) in Verbindung steht, wobei das Einlassmittel so eingerichtet ist, dass es das Fluid aus dem Behälter aufnimmt, und das weitere Auslassmittel mit dem vierten Lumen (38b) und mit dem Behälter (20) über ein Fluid in Verbindung steht,
wobei der weitere Ballon so groß ist, dass er, wenn er aufgeblasen wird, dehnbar ist, um die Innenfläche der Nasenhöhle zu bedecken,
und ferner umfassend
Druckregeldüsen (26a, 26b), die an den Röhren (16a, 16b) des Auslassmittels angrenzend an den Behälter angeordnet sind, wobei die Druckregeldüsen so eingerichtet sind, dass sie in den Röhren (16a, 16b) des Auslassmittels für Widerstand sorgen.

2. System nach Anspruch 1, wobei ein Temperaturregler (24) mit dem Behälter verbunden ist, wobei der Regler so eingerichtet ist, dass er die Temperatur des Fluids über eine Kühlvorrichtung regelt.

3. System nach Anspruch 1, wobei die Einlassmittel und Auslassmittel an einem proximalen Abschnitt (40, 40a, 40b) des Kathetermittels angeordnet sind.

4. System nach einem der vorhergehenden Ansprüche, wobei die Zirkulation des Fluids mittels hydrostatischen Drucks des Fluids in dem Behälter erreicht wird.

5. System nach einem der Ansprüche 1 bis 3, wobei das Mittel zum Zirkulierenlassen des Fluids ferner ein Pumpmittel (18) umfasst, das zwischen dem Behälter und dem Kathetermittel angeordnet ist.

6. System nach einem der vorhergehenden Ansprüche, wobei das Mittel zum Zirkulierenlassen des Fluids so eingerichtet ist, dass es für einen Durchfluss von ungefähr 25 - 1000 ml/min sorgt.

7. System nach einem der vorhergehenden Ansprüche, wobei die Druckregeldüsen einstellbar sind.

8. System nach Anspruch 1, ferner umfassend eine Temperaturüberwachungsvorrichtung (50), die so eingerichtet ist, dass sie indirekt oder direkt die Temperatur des Gehirns aufzeichnet und automatisch den Temperaturregler steuert, um die Temperatur des Fluids in dem Behälter über eine Wärmetauschervorrichtung zu regeln, um die Temperatur des Gehirns auf einem gewünschten Stand zu halten.

9. System nach Anspruch 8, wobei die Temperaturüberwachungsvorrichtung ein IR-Thermistor, eine MR-Vorrichtung, eine Nah-Infrarot-Vorrichtung oder eine Impedanzspektroskopie-Vorrichtung ist.

10. System nach Anspruch 8 oder 9, wobei die Temperaturüberwachungsvorrichtung dafür geeignet ist, an einer Stirn des Menschen oder in einem Gehörgang des Menschen angeordnet zu werden.

## Revendications

1. Système pour la régulation indirecte de la température par refroidissement du cerveau d'un être humain par le biais d'une cavité nasale de celui-ci, comprenant
un moyen de cathéter à lumière double (30a) adapté pour être introduit à travers une narine d'un être humain et placé avec son bout adjacent à l'arrière desdites cavités nasales,
ledit moyen de cathéter à lumière double comprenant une première lumière (32a) et une seconde lumière (38a), lesdites première et seconde lumières étant en communication fluidique au moyen d'un ensemble d'ouvertures (34a), ladite seconde lumière formant une membrane adaptée pour être agencée en contact avec une surface de ladite cavité nasale, ladite membrane définissant un ballonnet extensible ;
un moyen d'entrée pour introduire du fluide de refroidissement dans ledit ballonnet extensible ;
un moyen de sortie pour retirer du fluide de refroidissement dudit ballonnet extensible ; et
des moyens pour faire circuler ledit fluide de refroidissement dans ledit ballonnet extensible par le biais dudit moyen d'entrée et hors dudit ballonnet extensible par le biais dudit moyen de sortie, lesdits moyens comprenant un ensemble de tubes (12a, 16a) configuré pour relier ledit moyen de cathéter à un réservoir (20),
ledit moyen d'entrée étant en communication fluidique avec ledit réservoir (20) et avec ladite première lumière (32a), ledit moyen d'entrée étant configuré pour recevoir ledit fluide dudit réservoir, et ledit moyen de sortie étant en communication fluidique avec ladite seconde lumière (38a) et avec ledit réservoir (20),
ledit ballonnet présentant, quand il est gonflé, une dimension telle qu'il est extensible pour recouvrir la surface intérieure de la cavité nasale, et comprenant en outre un autre moyen de cathéter à lumière double (30b) adapté pour être introduit à travers une autre narine de l'être humain et placé avec son bout adjacent à l'arrière desdites cavités nasales,
ledit autre moyen de cathéter à lumière double (30b) comprenant une troisième lumière (32b) et une quatrième lumière (38b), lesdites troisième et quatrième lumières étant en communication fluidique au moyen d'un autre ensemble d'ouvertures (34b), ladite quatrième lumière formant une membrane adaptée pour être agencée en contact avec une autre surface de ladite cavité nasale, ladite membrane définissant un autre ballonnet extensible ;
un autre moyen d'entrée pour introduire du fluide de refroidissement dans ledit ballonnet extensible ;
un autre moyen de sortie pour retirer du fluide de refroidissement dudit ballonnet extensible ; et
un autre moyen pour faire circuler ledit fluide de refroidissement dans ledit ballonnet extensible par le biais dudit autre moyen d'entrée et hors dudit ballonnet extensible par le biais dudit autre moyen de sortie, ledit autre moyen comprenant un autre ensemble de tubes (12b, 16b) configuré pour relier ledit autre moyen de cathéter au réservoir (20),
ledit autre moyen d'entrée étant en communication fluidique avec ledit réservoir (20) et avec ladite troisième lumière (32b), ledit moyen d'entrée étant configuré pour recevoir ledit fluide dudit réservoir, et ledit autre moyen de sortie étant en communication fluidique avec ladite quatrième lumière (38b) et avec ledit réservoir (20),
ledit autre ballonnet extensible présentant, quand il est gonflé, une dimension telle qu'il est extensible pour recouvrir la surface intérieure de la cavité nasale,
et comprenant en outre
des buses de régulation de pression (26a, 26b) agencées sur lesdits tubes (16a, 16b) du moyen de sortie adjacentes audit réservoir, lesdites buses de régulation de pression étant configurées pour fournir une résistance dans lesdits tubes (16a, 16b) du moyen de sortie.

2. Système selon la revendication 1, dans lequel un régulateur de température (24) est relié audit réservoir, ledit régulateur étant configuré pour réguler la température dudit fluide, par le biais d'un dispositif de refroidissement.

3. Système selon la revendication 1, dans lequel lesdits moyens d'entrée et moyens de sortie sont agencés sur une partie proximale (40, 40a, 40b) du moyen de cathéter.

4. Système selon l'une quelconque des revendications précédentes, dans lequel ladite circulation dudit fluide est réalisée au moyen d'une pression hydrostatique dudit fluide dans ledit réservoir.

5. Système selon l'une quelconque des revendications 1 à 3, dans lequel ledit moyen pour faire circuler ledit fluide comprend en outre un moyen de pompage (18) agencé entre ledit réservoir et ledit moyen de cathéter.

6. Système selon l'une quelconque des revendications précédentes, dans lequel ledit moyen pour faire circuler le fluide est configuré pour assurer un débit d'approximativement 25 à 1000 ml/min.

7. Système selon l'une quelconque des revendications précédentes, dans lequel lesdites buses de régulation de pression sont réglables.

8. Système selon la revendication 1, comprenant en outre un dispositif de surveillance de la température (50) configuré pour, indirectement ou directement, enregistrer la température du cerveau et pour contrôler automatiquement ledit régulateur de température pour réguler la température dudit fluide dans ledit réservoir, par le biais d'un dispositif d'échange thermique, afin de maintenir la température du cerveau à un niveau souhaité.

9. Système selon la revendication 8, dans lequel ledit dispositif de surveillance de la température est une thermistance IR, un dispositif de résonance magnétique, un dispositif proche infrarouge ou un dispositif de spectroscopie d'impédance.

10. Système selon la revendication 8 ou 9, dans lequel ledit dispositif de surveillance de la température est adapté pour être agencé sur un front de l'être humain ou dans un canal auditif de l'être humain.
